(19) Europäisches Patentamt
European Patent Office
Office européen des brevets

(11) **EP 2 156 890 B1**

(12) **EUROPÄISCHE PATENTSCHRIFT**

(45) Veröffentlichungstag und Bekanntmachung des Hinweises auf die Patenterteilung:
**08.04.2015 Patentblatt 2015/15**

(51) Int Cl.:
***B01L 3/00*** *(2006.01)*    ***C12M 1/34*** *(2006.01)*

(21) Anmeldenummer: **09168042.1**

(22) Anmeldetag: **18.08.2009**

(54) **Anordnung und Verfahren zum Erzeugen, Manipulieren und Analysieren von Kompartimenten**

Assembly and method for generating, manipulating and analysing compartments

Agencement et procédé de production, de manipulation et d'analyse de compartiments

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.08.2008 DE 102008039117**

(43) Veröffentlichungstag der Anmeldung:
**24.02.2010 Patentblatt 2010/08**

(73) Patentinhaber: **INSTITUT FÜR BIOPROZESS- UND ANALYSENMESSTECHNIK E.V.**
**37308 Heiligenstadt (DE)**

(72) Erfinder:
• **Lemke, Karen**
  **37073 Göttingen (DE)**
• **Gastrock, Gunter**
  **37308 Heiligenstadt (DE)**
• **Grodrian, Andreas**
  **37308 Heiligenstadt (DE)**
• **Römer, Robert**
  **37308 Heiligenstadt (DE)**
• **Quade, Mandy**
  **07548 Gera (DE)**
• **Roscher, Dietrich**
  **98693 Ilmenau (DE)**

(74) Vertreter: **Liedtke, Klaus**
**Liedtke & Partner**
**Patentanwälte**
**Gerhart-Hauptmann-Strasse 10-11**
**99096 Erfurt (DE)**

(56) Entgegenhaltungen:
**WO-A1-2008/125347    DE-A1-102005 061 629**
**US-A1- 2005 172 476**

EP 2 156 890 B1

**Beschreibung**

Die Erfindung betrifft eine Anordnung und ein Verfahren zum Kompartimentieren und zum Manipulieren von Kompartimenten. Dabei werden stabile Mikrosphären aus einem gasförmigen oder flüssigen Medium erzeugt, die Probenmaterial wie beispielsweise anorganische und/oder organische Substanzen in beliebigen Aggregatzuständen, bioaktive Moleküle oder auch Zellen oder Mikroorganismen enthalten und die durch einen mit den Mikrosphären nicht mischbaren Transportfluid beispielsweise zum Zweck des Manipulierens, des Speicherns und des Auswertens transportiert werden. Probenmaterial und Mikrosphäre entsprechen dem Probenfluid; das Probenfluid und die Grenzfläche bzw. Phasengrenze zwischen Mikrosphäre und Transportfluid bilden das Kompartiment.

Durch das Vereinzeln der Zellen bzw. Mikroorganismen in jeweils einer eigenen Mikrosphäre werden Querkontaminationen ausgeschlossen, und es kann eine individuelle Behandlung mit jeweils speziellen Effektoren, aber auch beispielsweise Viren oder eine gezielte Selektion von einzelnen Kompartimenten erfolgen. Ein Kompartimentieren ist deshalb immer dann vorteilhaft, wenn eine große Anzahl von Ansätzen (Proben) notwendig ist, um eine Optimierung von Reaktionsansätzen zu erhalten oder um viele Faktoren unabhängig voneinander auf vergleichbare Kompartimente statistisch abgesichert zu testen. Ein Kompartimentieren ist aber auch dann vorteilhaft, wenn einzelne oder wenige Proben detektiert werden müssen. Die gasförmigen oder flüssigen Mikrosphären bleiben von der Umwelt abgeschlossen, so dass auch kein arbeitsschutztechnisches Risiko entstehen kann. Dadurch entstehen Vorteile gegenüber z. B. konventionellen Zellmanipulationssystemen, bei denen die Zellen direkt durch einen Trägerstrom transportiert werden.

Kompartimentiersysteme zur Erzeugung von Mikrosphären bzw. Probenfluiden sind bekannt. In der DE 101 45 568.2 wird ein Verfahren zur Kultivierung und Analyse von mikrobiellen Einzelzellkulturen beschrieben, das auf dem Kompartimentieren einer Nährstofflösung mit eingebrachten Kulturen beruht. Ein komplexes mikrofluidisches System wird in der Schrift US 2008/0003142 vorgestellt, das Bausteine zum Erzeugen von Kompartimenten, zum Zudosieren von Effektoren, zum Speichern und zum Sortieren enthält. Speziell mit dem Screening von Kompartimenten beschäftigt sich die US 2005/0221339.

Ein System zum Erzeugen von Kompartimenten ist auch in US-A-20050172476 beschrieben.

Von besonderer Bedeutung für die Funktion von derartigen Systemen ist das Kompartimentieren selbst. Da die eigentlichen Mikrosphären je nach Probenart nur im Durchmesser eine minimale Größe von 50 $\mu$m besitzen und zum Beispiel bei polaren Mikrosphären das Transportfluid aus unpolaren Fluiden, wie z.B. Tetradecan oder Ölen besteht, sollte beim Kompartimentieren jeder Kontakt zwischen organischer Probe und Transportfluid sowie mechanischer Stress vermieden werden. Verwendet werden dazu planare Mikrofluidik-Kanalsysteme mit T- oder Y- förmigem Layout, wie sie z.B. in der WO 2006/098700 oder DE 103 22 942 oder auch der bereits erwähnten US 2008/0003142 erläutert wurden.

Nachteilig bei der Verwendung dieser Art von Kanalsystemen ist, dass der Aufwand für die Herstellung der feinen Kanalstrukturen sehr hoch ist. Insbesondere bestehen extrem hohe Anforderungen an die Mikrogeometrie bei der Einmündung des Kanals für das Probenfluid in den Kanal mit dem Transportfluid, um ein funktionierendes Kompartimentieren zu ermöglichen. Vorzugsweise sollten die Mikrokanäle mit rundem Querschnitt ausgeführt werden, um einen stabilen Transport der Kompartimente durch den Mikrokanal ohne Änderung des Abstandes der Kompartimente zueinander zu gewährleisten. Die Herstellung runder Kanäle in einem planaren Mikrosystem ist zwar technisch möglich, stellt aber auch einen sehr aufwändigen technologischen Prozess dar.

Auch ist die Reinigung der im Mikrosystem eingebrachten Kanäle nahezu unmöglich, sodass notwendigerweise das gesamte planare Mikrosystem als Einwegprodukt betrachtet werden sollte.

Ein weiteres Problem bei der Verwendung der oben aufgeführten Kanalsysteme ist der Transport des Probenfluids aus einem Behälter (z. B. aus einem Well oder einem Bioreaktor) zur Kompartimentierstation. Der Transport des zu kompartimentierenden Probenfluids erfolgt durch Verbindungen wie Schläuche, Rohre, etc. Innerhalb dieser Verbindungen treten insbesondere für biologisches Probenmaterial nachteilige Effekte bedingt durch Temperatur- und Effektorgradienten, Sedimentationseffekte, Totvolumina sowie Adhäsionseffekte auf. Diese nachteiligen Effekte werden zudem durch eine geringe Fließgeschwindigkeit des zu kompartimentierenden Probenfluids begünstigt, wenn z.B. ein mechanischer Stress für Zellen vermieden werden soll.

Weiterhin sind Probeentnahmesonden bekannt, die aus zwei ineinander liegenden rohrförmigen Bauteilen bestehen und die in eine Probe eintauchen. Die DE 197 15 441 ein Verfahren zur In-situ-Kalibrierung, bei der die Probe durch das innere Rohr abgesaugt wird und einen Sensor kontaktiert. Durch das äußere Rohr wird eine Konditionierlösung oder auch eine Kalibrierlösung zugeführt. Im US Patent 5,191,900 wird ebenfalls eine doppellumige Sonde beschrieben, die als Dialysesonde eingesetzt. Die beschriebenen Verfahren bzw. Anordnungen sind jedoch nicht für die Erzeugung bzw. die Manipulation von Kompartimenten ausgestaltet.

Aufgabe der Erfindung ist es, eine Anordnung und ein Verfahren für das Kompartimentieren, das Kultivieren, das Manipulieren in und mit den Kompartimenten, das Speichern und das Detektieren/Analysieren der Kompartimente, insbesondere von in den Kompartimenten enthaltenem Probematerial unter Vermeidung der oben genannten Nachteile vorzuschlagen.

Die Aufgabe wird durch die Verwendung von ineinander geschachtelten Fluidleitern zum Ausbilden voneinander getrennter Fluidpfade gelöst, wobei jedem Fluidleiter durch eine steuerbare Flussquelle ein Fluss eines bestimmten Fluids aufgeprägt wird, und die Fluidleiter mit dem anderen Ende in ein Fluidreservoir, z.B. ein Well mit einem weiteren Fluid eintauchen. Weitere Merkmale der vorteilhaft ausgestalteten Erfindung sind den Unteransprüchen zu entnehmen.

Mit dieser Anordnung ist es möglich, je nach Ausführung des Wells, Richtung und Größe der aufgeprägten Fluide und Art der Fluide alle Funktionen eines Kompartimentiersystems wie beispielsweise Kompartimentieren, Dekompartimentieren, Selektieren, Zusammenführen verschiedener Kompartimente, Splitten von Kompartimenten und Zudosieren von Fluidaliquots durchzuführen.

In einer vorteilhaften Ausgestaltung der Erfindung werden die Fluidleiter in einer Aufsetzsonde untergebracht, deren Merkmale den Einsatz für alle der genannten Funktionen eines Kompartimentiersystems mit einer einzigen Ausführungsform ermöglichen. Der Aufbau der erfindungsgemäßen Anordnung ist sehr einfach und robust, da die für die Funktionsweise des Kompartimentierens wichtigen Fluidleiter aus Kanülen und Schläuchen hergestellt werden, die als Massenware mit hoher Präzision und niedrigen Kosten für unterschiedlichste Anwendungen hergestellt werden. Die Nachbearbeitung der Kanülen wie Ablängen, Erzeugen von Planflächen und Beschichtung ist einfach mit konventionellen Fertigungsverfahren zu bewerkstelligen.

Die erfindungsgemäße Anordnung ist in ihrer vorteilhaften Ausgestaltung in Modulen aufgebaut und leicht demontierbar und montierbar, sodass ein Reinigen bzw. Sterilisieren sowie der Austausch von Schläuchen einfach möglich ist. In einem Ausführungsbeispiel wird die Sondenanordnung nach jedem Eintauchvorgang mit einem Reinigungsfluid gespült, das durch einen weiteren Fluidleiter konzentrisch um die Sondenanordnung geleitet wird.

Für die Funktion der erfindungsgemäßen Anordnung sind steuerbare Flussquellen (Fluss = Volumen pro Zeiteinheit) notwendig, die in einer Analogie mit elektrischen Stromquellen vergleichbar sind. Steuerbare Flussquellen sind z. B. Pumpen, deren Förderleistung in einem definierten Betriebsbereich unabhängig vom Gegendruck der angeschlossenen Fluidleiter ist. Spritzenpumpen, bei denen der Kolben z.B. durch einen Schrittmotor angetrieben wird, erfüllen in erster Näherung diese Anforderungen. Aber auch Pumpen, deren Förderleistungen stark vom Gegendruck abhängig sind, wie z. B. Schlauch- oder Zahnradpumpen, lassen sich durch Einbindung in einen Regelungskreis mit einem Flussmesser als Flussquellen betreiben.

Für den Betrieb der erfindungsgemäßen Anordnung sind verschiedene Betriebszustände der Fluidleiter notwendig. Der Betriebszustand f = Fi (i = 1... n) bedeutet, dass dem Fluidleiter ein definierter Fluss in einer definierten Richtung aufgeprägt wird. Die Größe des Flusses ist vom Strömungswiderstand des Kanalsystems und einem eventuell vorhandenen Gegendruck unanhängig. Im Betriebszustand f = 0 wird im Fluidleiter jeder Fluss z.B. durch ein geschlossenes Ventil gesperrt. Im Betriebszustand f = nn wird ein Fluss im betreffenden Fluidleiter freigegeben, d.h. der sich einstellende Fluss hängt von Druckdifferenzen im Fluidleiter ab. Beim Kompartimentieren können die eingebundenen Flussquellen kontinuierlich oder alternierend arbeiten.

Zum Zweck des Kompartimentierens tauchen die ineinander geschachtelten Fluidleiter in einen Behälter, der mit Probenfluid gefüllt ist, wobei im Raum zwischen dem inneren und äußeren Fluidleiter ein mit dem Probenfluid nicht mischbares Transportfluid in den Behälter mit Probenfluid mit einem Volumenstrom Φ2 gedrückt wird, gleichzeitig aber durch den inneren Fluidleiter Transportfluid mit eingeschlossenen Kompartimenten als Kompartimentenstrom mit einem Fluss Φ1 > Φ2 abgesaugt wird.

Auf der Grundlage der erfindungsgemäßen Anordnung ist das Erzeugen von Kompartimenten mit eingeschlossenem organischen und/oder anorganischen Material sowie mittels beliebiger Verbindungen erzeugter Komplexe bestehend aus organischen und anorganischen sowie mittels beliebiger Verbindungen erzeugter Komplexe bestehend aus nur anorganischen oder nur organischen Material auf besonders schonende Art und Weise möglich. Die Entnahme von Proben aus einem Probenfluid, das organisches Material wie beispielsweise eukaryotische Zellen (unerheblich ob adhärent auf einem Mikrocarrier oder in Suspension), Zellgewebe, Sphäroide, Einzeller, Mehrzeller, Pilze, Viren, Bacteriophagen, Mikroorganismen, Organellen etc. beinhaltet, erfolgt ohne mechanischen Stress. Damit ist die Überlebensfähigkeit des organischen Materials während des Kompartimentierens und im Anschluss daran sehr hoch. Ebenso vermeidet die schonende Art und Weise des Kompartimentierens das durch mechanischen Stress hervorgerufene Trennen der Verbindungen zwischen den Komplexkomponenten. Das Kompartimentieren erfolgt quasi totvolumenfrei in dem im Well befindlichen Probenfluid am Übergang zwischen dem Probenfluid und dem inneren Fluidleiter. Das Kompartiment kann anschließend, in das Transportfluid eingebettet, auch über lange Transportwege zur Detektion, beispielsweise in kommerziellen Analysatoren, geleitet werden.

Für die Erfindung ist es unerheblich, welcher Art das Pobenmaterial ist. Für die Erfindung ist es weiterhin unerheblich, ob sich das Probenmaterial in der Mikrosphäre oder an bzw. in der Grenzfläche/Phasengrenze zwischen Mikrosphäre und Trägermedium befindet, solange das Probenmaterial ohne zusätzlich von außerhalb aufgeprägte Kräfte über diese Grenzfläche/Phasengrenze nicht in das Transportfluid gelangen kann.

Im Vergleich zum Kompartimentieren mittels planarer Mikrofluidik- Kanalsysteme mit T- oder Y- förmigem Layout hat das Kompartimentieren auf der Grundlage der erfindungsgemäßen Anordnung die Vorteile, dass keine Totvolumina vorhanden sind und entstehen können, dass keine Temperatur- und Effektorgradienten entstehen sowie keine Sedimentations- und Adhäsionseffekte auftreten können. Jegliche Konditionierung des Probenfluids ist mit einfachen Mitteln und Verfahren (Mischen, Temperieren etc.) möglich, was z. B. eine reproduzierbare Verteilung des Materials in den Kompartimenten sichert. Durch Parametervariation der aufgeprägten Flüsse ergibt sich ein großes Funktionsfenster, dass beispielsweise bei wässrigen Probenfluids die Auswahl unterschiedlicher Transportfluide wie beispielsweise Tetradekan, Öle, Perfluorcarbone (unerheblich ob begast oder unbegast), Gase zulässt.

Beim Dekompartimentieren wird der Kompartimentenstrom in einen Behälter abgegeben. Dabei erfolgt eine Auftrennung des Transportfluids und des kompartimentierten Probenfluids. Die gleiche Anordnung ineinander verschachtelter Fluidleiter wird auch, wie in den Ausführungsbeispielen beschrieben wird, zum Zudosieren von Effektoren in das Kompartiment, zur Selektion von Kompartimenten, zum Teilen (Splitting) von Kompartimenten sowie zum Vereinigen von Kompartimenten verwendet. Die dazu verwendeten Behälter, beispielsweise Wells, besitzen eine unterschiedliche Ausführung und können, wie in einem Ausführungsbeispiel der Erfindung erläutert wird, in einem Tray zusammengefasst werden. In der Kombination verschiedener Arbeitsstationen entsteht ein Laborgerät, in dem Prozesse wie beispielsweise des Konditionierens, der chemischen, biologischen und/oder physikalischen Reaktion, des Kultivierens, des Speicherns und des Analysierens wie auch das Manipulieren im und mit dem Kompartiment voll automatisiert ablaufen.

Die Erfindung soll an Ausführungsbeispielen erläutert werden:

Es zeigen

Fig. 1a:  Anordnung für ineinander verschachtelte Fluidleiter für $\Phi2 = \Phi1$

Fig. 1b:  Anordnung für ineinander verschachtelte Fluidleiter für $\Phi2 = 0$; $\Phi1 > 0$

Fig. 2:  Phasendarstellung des Kompartimentiervorganges

Fig. 3a:  Phasendarstellung des Kompartimentiervorganges zum Zeitpunkt des Transportfluideintritts in den Fluidleiter 1 als Draufsicht entsprechend Schnitt A - A in Fig. 2

Fig. 3b:  Phasendarstellung des Kompartimentiervorganges zum Zeitpunkt des Pro- benfluideintritts in den Fluidleiter 1 als Draufsicht entsprechend Schnitt A - A in Fig. 2

Fig. 4:  Vorgang des Dekompartimentierens

Fig. 5a:  Schema der Aufsetzsonde beim Aufsetzen

Fig. 5b:  Schema der Aufsetzsonde im eingetauchten Zustand

Fig. 6:  Spülen mit Aufsetzsonde im Well

Fig. 7:  Draufsicht auf Sondenanschluss entsprechend Schnitt B - B in Fig. 6

Fig. 8:  Selektionsanordnung mit drei Aufsetzsonden

Fig. 9:  Zusammenführung von Kompartimentenströmen

Fig. 10:  Teilen von Kompartimenten

Fig. 11:  Zudosieren von Effektoren in Kompartimente nach dem Stand der Technik

Fig. 12 :  Zudosieren von Kompartimenten unter Verwendung ineinander geschachtelter Fluidleiter

Fig. 13:  Effektorzugabe mit Aufsetzsonde

Fig. 14:  Synchronisationsanordnung für Effektorzugabe

Fig. 15:  Elektrodenanordnung

Fig. 16:  Laborsystem

Fig. 17:  Regelkreis für Schlauchpumpen

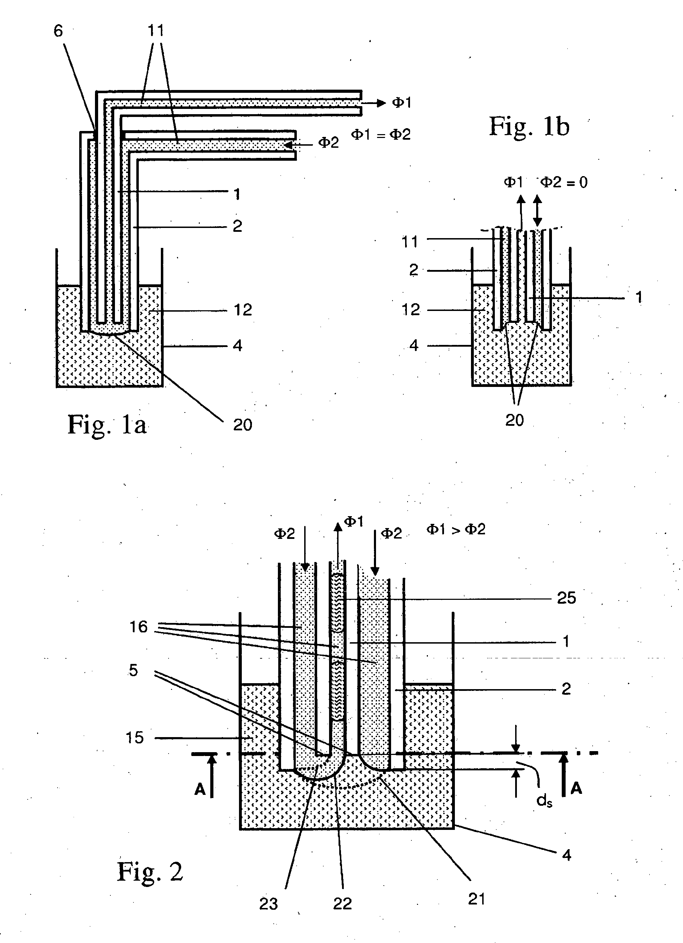

Um den Vorgang des Kompartimentierens zu erläutern, sollen zunächst zwei Grenzfälle in Fig. 1a und Fig. 1b betrachtet werden. Fig. 1a und Fig. 1b zeigen das Aufbauschema einer Sondenanordnung mit zwei ineinander geschachtelten Fluidleitern 1 und 2, die mit ihren gemeinsamen Enden in ein Well 4 eintauchen. Eine Rohrdurchführung 6 sorgt dafür, dass getrennte Anschlüsse für die Fluidleiter 1 und 2 vorhanden sind.

In der Fig. 1a ist dargestellt, dass sich im Well 4 beispielsweise ein polares Fluid 12, wie z.B. Wasser befindet. In den Fluidleiter 2 wird ein unpolares Fluid 11 z. B. Tetradecan mit der Flussrate (Volumen pro Zeiteinheit) $\Phi2$ hineingedrückt, aus dem Fluidleiter 1 wird mit der gleichen Flussrate $\Phi1 = \Phi2$ das unpolare Fluid 11 abgesaugt. Wenn $\Phi1 = \Phi2$ ist, bildet sich an den in das polare Fluid 12 ragenden Enden der Fluidleiter 1 und 2 zwischen dem polaren Fluid 12 und dem unpolaren Fluid 11 eine Grenzfläche in Form eines stabilen Probenmeniskus 20 aus. Ausschließlich das durch den Fluidleiter 2 in Richtung Well 4 gepumpte unpolare Fluid 11 wird durch den Fluidleiter 1 aus Richtung Well 4 vollständig abgesaugt, das polare Fluid 12 verbleibt im Well 4.

In Fig. 1b wird der Fall gezeigt, dass nur durch den Fluidleiter 1 Fluid 12 mit einer definierten Flussrate Φ1 entnommen wird. Der Fluidleiter 2 ist gesperrt Φ2 = 0). In diesem Fall wird das polare Fluid 12 aus dem Well 4 durch den Fluidleiter 1 abgesaugt. Der Probenmeniskus 20 schließt das unpolare Fluid 11 im Fluidleiter 2 gegenüber dem polaren Fluid 12 im Well 4 ab.

In Fig. 2 wird der Vorgang des Kompartimentierens verdeutlicht. Zu diesem Zweck wird in den Fluidleiter 2 ein unpolares Transportfluid 16 mit der Flussrate Φ2 in Richtung Well 4 hineingedrückt, wobei ein hier nicht dargestelltes Pumpensystem verwendet wird, dessen Kennlinie einen vom Gegendruck des Fluidpfades im Fluidleiter 2 relativ konstanten Flussrate Φ2 aufprägt. Dadurch dehnt sich zunächst der Probenmeniskus auf Position 21 aus. Durch Absaugen des unpolaren Transportfluids 16 durch den Fluidleiter 1 mit einem Fluss Φ1 > Φ2 tritt eine Verarmung an Transportfluid 16 an der Stirnfläche 5 des Fluidleiters 1 ein. Der Probenmeniskus springt zu einer neuen Position 22. Ein weiteres Absaugen des Transportfluids 16 durch den Fluidleiter 1 bewirkt schließlich, dass der Probenmeniskus die Position 23 einnimmt, wodurch die Öffnung im Fluidleiter 1 für das Probenfluid 15, bestehend beispielsweise aus organischen Material und einem polaren Fluid, freigegeben wird. Das Probenfluid 15 tritt in den Fluidleiter 1 ein. Damit entsteht nunmehr wieder ein Überschuss an Transportfluid 16 im Bereich der Stirnfläche 5 des Fluidleiters 1, der schließlich zum Umspringen des Probenmeniskus von der Position 23 in die Position 22 führt.

Dieser geschilderte Vorgang wiederholt sich periodisch, wobei das Verhältnis der Länge der im Fluidleiter 1 gebildeten Kompartimente 25 zu ihrem Abstand wesentlich durch das Verhältnis der beiden Flussraten Φ1 zu Φ2 bestimmt wird. Weiteren Einfluss auf dieses Verhältnis haben die geometrischen Kenngrößen der Fluidleiter 1 und 2 sowie deren Oberflächenenergien ebenso wie die Oberflächenenergien der verwendeten Fluide. Vorteilhaft für das Kompartimentieren ist ein Versatz x des inneren Fluidleiters 1 zum äußeren Fluidleiters 2 um den Betrag x, wie in Fig. 2 eingezeichnet.

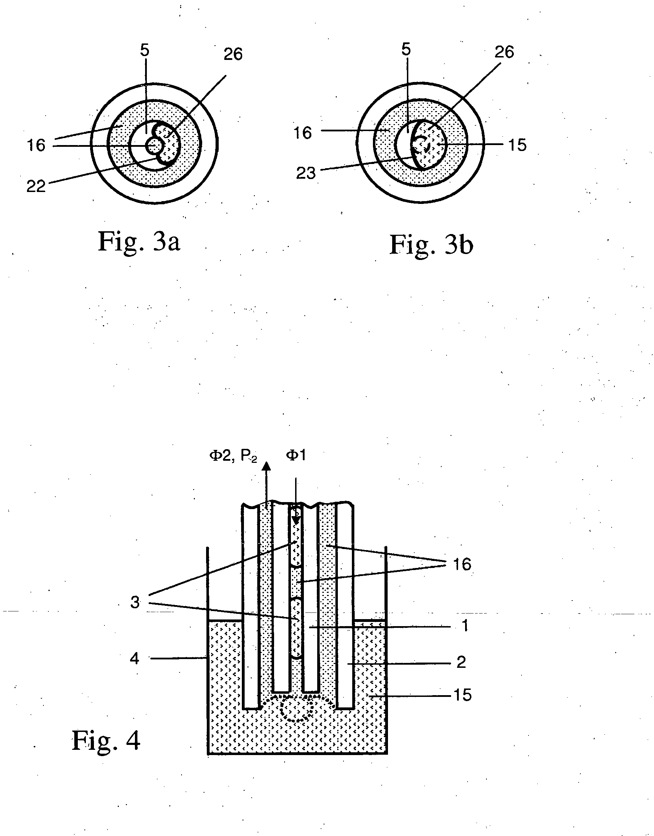

Die Funktionsweise des Kompartimentiervorganges wird anhand der Fig. 3a und 3b als Draufsicht auf den Schnitt A-A in Fig. 2 zusätzlich verdeutlicht. In der Meniskusposition 22 wird die Stirnfläche 5 durch das Probenfluid 15 nur teilweise bedeckt. Die Öffnung im Fluidleiter 1 ist für das Transportfluid 16 freigegeben. Da mehr Transportfluid 16 durch den Fluidleiter 1 abgesaugt wird, als durch den Fluidleiter 2 nachströmt, zieht sich der Meniskus auf die Meniskusposition 23 zurück. Damit erhöht sich der Grad der Bedeckung von Stirnfläche 5 (Fig. 3b), was dazu führt, dass die Öffnung des Fluidleiters 1 für das Einsaugen des Probenfluids 15 freigegeben wird.

Für die Funktionsweise des Kompartimentierens ist die Ausbildung der Stirnfläche 5 an der Spitze des Fluidleiters 1 bedeutsam, da das Rückspringen des Probenmeniskus von der Position 23 in die Position 22 davon abhängig ist, dass das polare Probenfluid 15 durch das unpolare Transportfluid 16 von der Stirnfläche 5 verdrängt wird. Dafür ist eine hydrophobe Beschichtung der Stirnfläche 5 vorteilhaft. Weiterhin ist ein etwa doppelt so großer Außendurchmesser des Fluidleiters 1 im Verhältnis zu seinem Innendurchmesser vorteilhaft, damit eine ausreichend große Stirnfläche 5 zur Ausbildung von zwei unterschiedlichen, energetisch stabilen Zuständen befördert wird.

Bei idealer Symmetrie wäre die Position des Bedeckungsfleckes nach Fig. 3a und 3b invariant, dass heißt, der Bedeckungsfleck 26 würde auf der Stirnfläche 5 beliebige Positionen einnehmen können, was unter Umständen zu einem periodisch instabilen Kompartimentiervorgang führt. Zur Vermeidung dieser Positionsinstabilität des Bedeckungsfleckes 26 ist es von Vorteil, eine reproduzierbares energetisches Minimum der gesamten Anordnung, insbesondere im Bereich der Kompartimententstehung zu erzeugen. Das kann durch Gradienten der Geschwindigkeit des Volumenstromes des Transportfluids 16 über die Querschnittsfläche zwischen Fluidleiter 1 und Fluidleiter 2 erreicht werden. Weitere Möglichkeiten für die Herstellung eines energetischen Minimums sind beispielsweise geometrische und/oder oberflächenenergetische Modifizierungen im Bereich der Kompartimententstehung. Diese Modifizierungen führen zu einem geometrisch definierten und reproduzierbaren Strömen des Transportfluids 16 über die in diesem Falle hydrophobe Stirnfläche 5 in den Fluidleiter 1. Damit ist gleichzeitig die geometrische Position des Bedeckungsfleckes 26 auf der Stirnfläche 5 energetisch definiert und somit die Voraussetzung für ein reproduzierbares Kompartimentieren erfüllt.

Die Erzeugung des reproduzierbaren energetischen Minimums im Bereich der Kompartimententstehung ist mittels verschiedener Ausbildungen möglich, wie z.B. partielle hydrophobe und hydrophile Beschichtung der Stirnfläche 5, Abweichungen der Symmetrieachse des Fluidleiters 1 von der Symmetrieachse des Fluidleiters 2, Neigung der Stirnfläche 5 relativ zur Achse des Fluidleiters 1 sowie lanzettförmiger Anschliff des Fluidleiters 2. Weiterhin ist es auch möglich, beispielsweise mit Hilfe akustischer und/oder elektromagnetischer Wellen Energieminima zu erzeugen, die ein reproduzierbares Kompartimentieren erlauben.

Bei dem im Zusammenhang mit den Erläuterungen zu den Fig. 1 bis 3 dargestellten Ausführungsbeispiel ist von einem polaren Probenfluid 15 und einem unpolaren Transportfluid 16 ausgegangen worden. Es liegt im Wesen der Erfindung, das sich Kompartimentiervorgänge mit anderen Kombinationen der Fluide (Flüssigkeiten und Gase) mit unterschiedlichen polaren und unpolaren bzw. nicht miteinander mischbaren Eigenschaften ebenso realisieren lassen. Wichtig ist dabei nur, dass zwei Fluide unterschiedlichen Typus als Ausgangsfluide für das Kompartimentieren verwendet werden und dass wenigstens die Oberflächen des Fluidleiters 1 gute Benetzungseigenschaften für das Transportfluid 16 besitzen bzw. antibenetzend für das Probenfluid 15 ausgeführt sind. Durch Parametervariation der aufgeprägten Flussraten $\Phi 1$ und $\Phi 2$ ergibt sich ein großes Funktionsfenster, dass die Auswahl unterschiedlicher Transport- und Probenfluide wie beispielsweise Alkane, Öle, Perfluorcarbone, wässrige Lösungen (alle Lösungen begast oder unbegast) oder Gase zulässt.

In Umkehrung des Prinzips des Kompartimentierens lassen sich Kompartimente eines Kompartimentenstroms 3 auch wieder in ein Probenfluid 15 zurückbringen, wie in Fig. 4 gezeigt wird. In diesem Fall wird der Kompartimentenstrom 3 (in Zeichnung nur zwei Kompartimente markiert) durch den Fluidleiter 1 zum Well 4 gefördert. Die Kompartimente des Kompartimentenstroms 3 vermischen sich mit dem Probenfluid 15. Durch eine auf den Fluidleiter 2 wirkende Saugkraft $P_2$ wird das mit dem Probenfluid 15 nicht mischbare Transportfluid 16 durch den Fluidleiter 2 abgesaugt. Alternativ kann auch bei bekannter Flussrate $\Phi 1$ des Kompartimentenstroms 3 auf den Fluidleiter 2 ein entsprechender Fluss $\Phi 2$ mit $\Phi 2 < \Phi 1$ aufgeprägt werden, dessen Verhältnis zu $\Phi 1$ eine vollständige Entsorgung des Transportfluids 16 aus dem Well 4 bewirkt. Auch beim Dekompartimentieren ist eine Abstimmung der verwendeten Fluide 15 und 16 auf die Oberflächeneigenschaften mindestens des Fluidleiters 1 als auch der Stirnfläche 5 wichtig. Bei einem unpolaren Transportfluid 16 ist deshalb beispielsweise die Wandung des Fluidleiters 1 mit einer hydrophoben bzw. oliophilen Oberfläche zu versehen.

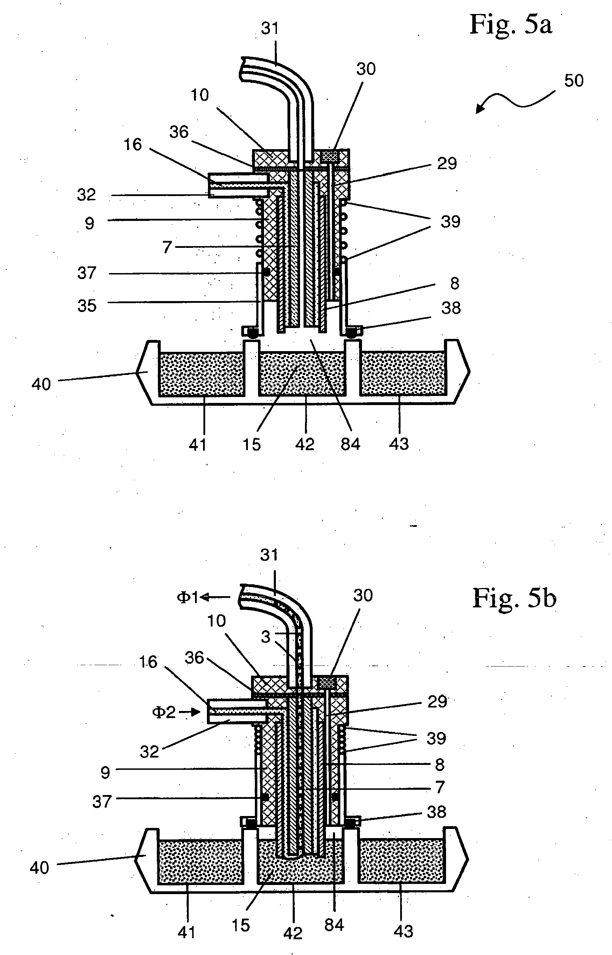

In Fig. 5a und Fig. 5b ist als beispielhafte Ausführung der Erfindung eine komplette Anordnung zum Kompartimentieren und Dekompartimentieren in Form einer Aufsetzsonde 50 dargestellt. Der innere Fluidleiter 1 besteht aus einem PTFE Schlauch 7 mit einem Innendurchmesser von 500 $\mu$m und einem Außendurchmesser von 1,6 mm. Dadurch wird eine für das Kompartimentieren wichtige Kreisringfläche als Stirnfläche 5 gebildet und können Mikrosphären bzw. Kompartimente mit einem Durchmesser von > 500 $\mu$m erzeugt werden.

PTFE weist typisch hydrophobe Eigenschaften auf, was den geforderten Eigenschaften für die Ausbildung der Stirnfläche 5 für den Fall entspricht, dass das Probenfluid 15 aus einer wässrigen Lösung besteht. Der PTFE-Schlauch 7 wird nach Zuschneiden auf Länge in eine gestufte Bohrung des Sondenkörpers 9 gesteckt. Der Fluidleiter 2 wird vorteilhaft aus einer Edelstahlkanüle 8 mit dem Innendurchmesser beispielsweise von 2 mm gefertigt, auf den PTFE-Schlauch 7 geschoben und im Sondenkörper 9 befestigt. Dabei ist es nicht von Nachteil, wenn das Ende des PTFE-Schlauches 7 nicht genau zentrisch innerhalb der Kanüle sitzt, da auch dadurch eine stabile Lage des Bedeckungsfleckes 26 gewährleistet werden kann.

Der Sondenkörper 9 enthält weitere Bohrungen für den Anschluss des Schlauches 32, über den das Transportfluids 16 geleitet wird. Die Zuführung bzw. Ableitung des Kompartimentstroms 3 (siehe Fig. 5a) erfolgt über einen zentralen Schlauchanschluss 31 im Sondenanschluss 10. Der Sondenanschluss 10 wird über die Dichtplatte 36 mit dem Sondenkörper 9 verbunden. Zusammen mit dem Well 42, in dem sich beispielsweise das Probenfluid 15 befindet, und der Hülse 35 wird beim Aufsetzen des Sondenkörpers 9 (siehe Fig. 5b) mit Hilfe der Führungsdichtung 37 und der Aufsetzdichtung 38 eine hermetisch abgeschlossene Kammer 84 gebildet, die für unterschiedliche, nachfolgend beschriebene Anwendungen genutzt werden kann. Die Hülse 35 wird durch eine Spiralfeder 39 in Richtung Well 42 gedrückt, sodass eine hermetisch abgeschlossene Kammer 84 bereits dann vorhanden ist, bevor der PTFE-Schlauch und die Kanüle bereits in die Probenflüssigkeit 15 eingetaucht ist.

Um während des Aufsetzens und Kompartimentierens Druckveränderungen in der hermetisch abgeschlossenen Kammer 84 auszugleichen, erhält der Sondenkörper 9 und der Sondenanschluss 10 einen Belüftungskanal 29, der am Sondenanschluss 10 mit einem Filter 30 abgeschlossen wird. Das Filter 30 kann beispielsweise ein Sterilfilter zur Verhinderung von Kontaminationen sein. Ebenso kann über das Filter 30 und den Belüftungskanal 29 die beim Aufsetzen des Sondenkörpers 9 entstandene Kammer 84 mit einem beliebigen Gas konditioniert werden. Das kann beispielsweise zur Kultivierung anaerober Mikroorganismen ein nicht Sauerstoff enthaltendes Gas oder Gasgemisch sein. Fig. 5b zeigt die Sonde im aufgesetzten Zustand im Betriebszustand des Kompartimentierens mit $\Phi 1 > \Phi 2$.

In einem Tray 40 (Fig. 5a, 5b) können verschiedene Wells, beispielsweise drei Wells 41 bis 43 mit verschiedenen Probenfluiden 15 nacheinander durch die Aufsetzsonde 50 angefahren werden. Es entsteht so ein Kompartimentenstrom 3, bei dem die einzelnen Kompartimente des Kompartimentenstroms 3 unterschiedliches organisches Material enthalten und eine Vermischung untereinander durch das Transportfluid 16 verhindert wird. Mehrere Wells können aber auch dazu benutzt werden, ein bestimmtes Well zu füllen, während das Probenfluid 15 eines anderen Wells gerade kompartimentiert wird. Viele Kombinationen sind hier denkbar. Wells können beispielsweise auch mit Rühreinrichtungen versehen werden, um eine gleichmäßige Probensuspension während des Kompartimentierens zu gewährleisten.

Weiterhin kann ein Well einen Bioreaktor beliebigen Volumens darstellen, wobei die Aufsetzsonde 50 an einer geeigneten Position am Bioreaktor aufgesetzt ist oder fest mit dem Bioreaktor verbunden ist. Aus der im Bioreaktor befindlichen Probensuspension können beliebig viele Kompartimente als repräsentative Proben entnommen werden, beispielsweise zum Zweck der Analyse.

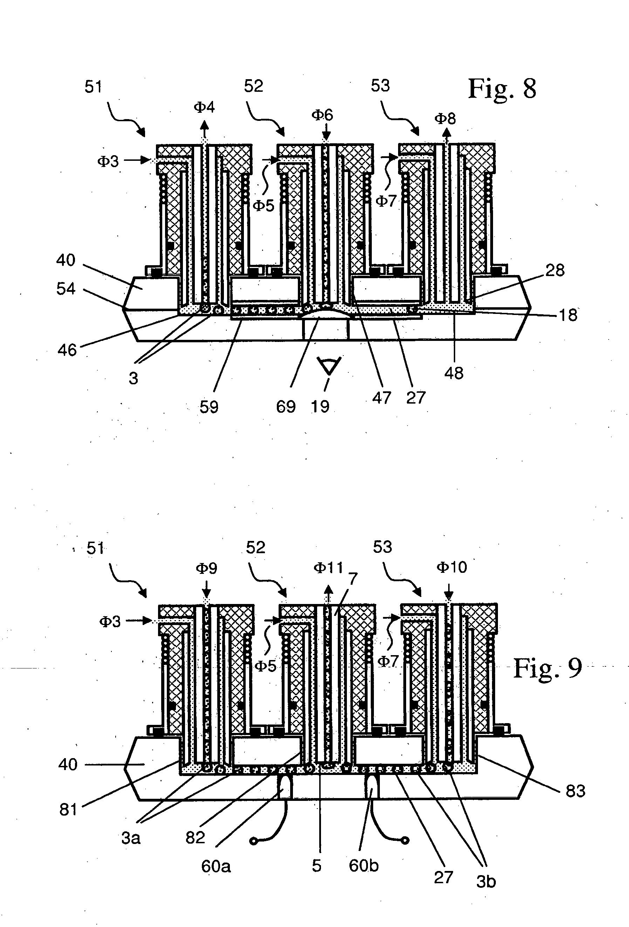

Ebenso ist es möglich, nur ein Kompartiment zu entnehmen und dieses im Transportfluid beispielsweise zu einem geeigneten Analysator zu transportieren. Dieser Analysator kann beispielsweise ein Mikroskop sein. Diese Anordnung stellt eine kostengünstige Alternative zur *in-situ*-Mikroskopie dar. Die Aufsetzsonde 50 ist aus dampfsterilisierbaren Materialien gefertigt und kann somit gemeinsam mit dem Bioreaktor und beispielsweise einer Fließzelle für die *in-situ*-Mikroskopie (vgl. Fig. 8) z.B. im Autoklaven oder *in situ* sterilisiert werden.

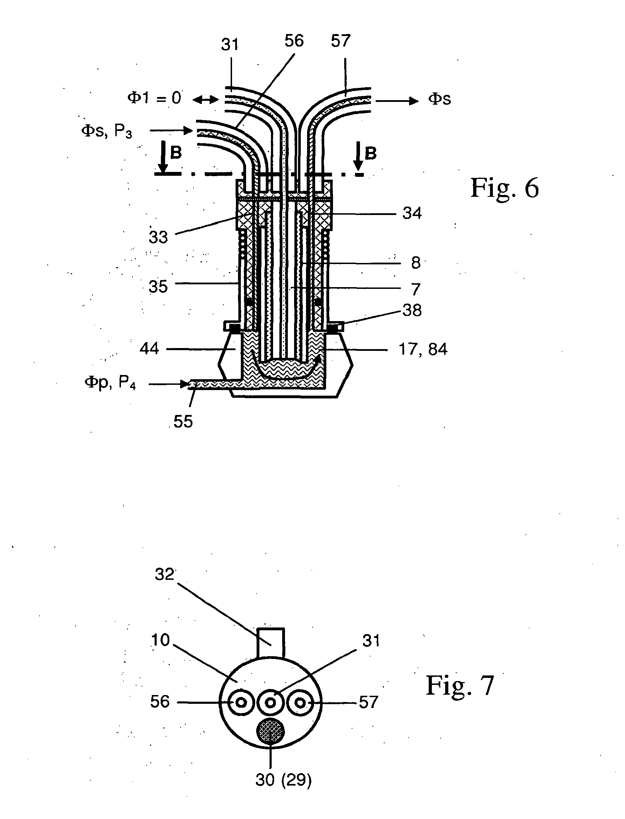

Für den Fall, dass durch die Aufsetzsonde 50 nacheinander verschiedene Probenfluide 15 aus den Wells 41, 42, 43 kompartimentiert werden, ist ein dazwischen liegender Spülvorgang, der zumindest die Außenflächen der Kanüle 8 erfasst, notwendig, um Querkontaminationen zu vermeiden. In Fig. 6 ist zu diesem Zweck ein weiterer Fluidpfad für Spülfluid 17 in der Aufsetzsonde 50 vorgesehen, der aus dem Schlauchanschluss 56, dem Spülkanalzulauf 33, der durch die Hülse 35 und dem Well 44 gebildeten Kammer 84 sowie dem Spülkanal 34 und dem Schlauchanschluss 57 für den Ablauf besteht. Je nach den Druckverhältnissen P3 und P4 wird entweder nur die Außenfläche der Kanüle 8 oder auch bei fehlendem Gegendruck auch der als PTFE-Schlauch 7 ausgeführte Fluidleiter 1 und/oder der als Kanüle 8 ausgeführte Fluidleiter 2 gespült. Dieser Fluidpfad für das Spülfluid 17 kann durch weitere Fluidpfade mit der Flussrate Fp ergänzt oder kombiniert werden, die durch einen Anschluss 55 für einen peripheren Fluidleiter entstehen.

Der für die Spülung mit Spülfluid 17 vorgesehene Fluidpfad kann auch in einer weiteren Ausgestaltung der Erfindung anderweitig zum Zuleiten eines beliebigen Gases oder Gasgemisches durch den Zulauf 56 und den Spülkanal 33 in die beim Aufsetzen des Sondenkörpers 9 entstehende Kammer 84 genutzt werden. Das Gas oder Gasgemisch wird dabei beispielsweise zur Konditionierung der Probenfluid 15 verwendet. Für diese Anwendung wird entweder eine nur für diesen Zweck eingesetzte baugleiche Aufsetzsonde 50 verwendet oder es erfolgt ein Umschalten zwischen Spülfluid 17 und dem Gas bzw. Gasgemisch mittels geeigneter Ventile (nicht dargestellt).

Das für die Spülung vorgesehene Well 44 kann ebenfalls zusammen mit dem, ein Probenfluid 15 enthaltenen Well 41, 42, 43 auf einem gemeinsamen Tray 40 untergebracht werden. Die Anordnung der verschiedenen Schlauchanschlüsse 31, 32, 56, 57 für Probenfluid 15, Transportfluid 16 bzw. Spülfluid 17 verdeutlicht Fig. 7 als Draufsicht auf den Sondenanschluss in Fig. 6 im Schnitt B-B, wobei unterschiedliche radiale Ebenen zur Unterbringung der Spülkanäle 33 und 34, des Belüftungskanals 29 bzw. des Filters 30 sowie der Schlauchanschlüsse 31, 32, 56, 57 im Sondenkörper 9 bzw. Sondenanschluss 10 genutzt werden.

In einer weiteren Ausgestaltung der Erfindung nach Fig. 8 werden mindestens drei Wells 46, 47, 48 angeordnet, die durch Verbindungskanäle 27 miteinander verbunden sind. Bei dieser Anordnung werden aus dem durch die mittlere Aufsetzsonde 52 gelieferten Kompartimentenstrom 3 einzelne Kompartimente 18 nach einer Auswertung selektiert. Beispielsweise können optische Auswerteverfahren verwendet werden, wobei die Optik 69 hier beispielsweise als Mikroskop ausgeführt ist und die Auswertung 19 als visuelle Beobachtung oder auch mit Hilfe einer Kamera, die über eine Bildverarbeitung Selektionsmerkmale ermittelt, erfolgt. Das heißt, dass die Well Selektionszuführung 47 für die Ermittlung eines Selektionskriteriums zur Aufteilung des Kompartimentenstroms 3 eine aus der Optik 69 und Auswerteeinheit 19 gebildete Analysevorrichtung enthält, die zur Ermittlung des Zustandes der einzelnen Kompartimente verwendet wird.

Durch Steuerung der Flüsse Φ3 und Φ4 bzw. Φ7 und Φ8 kann in der Anordnung nach Fig. 8 entschieden werden, ob ein selektiertes Kompartiment 18 die in der Fig. 8 dargestellte linke Aufsetzsonde 51 oder die rechte Aufsetzsonde 53 wieder verlässt. Es entspricht der erfindungsgemäßen Anordnung, wenn statt zwei Aufsetzsonden 51 oder 53 konzentrisch um das Well 47 bzw. um die Aufsetzsonde 52 mehr als zwei z.B. acht Wells entsprechend acht zu selektierender Gruppen von Kompartimenten angeordnet werden.

Die zwischen den Wells des Tray 40 vorhandenen Verbindungskanäle 27 werden in einem Ausführungsbeispiel als Koppelschläuche 59 aus PTFE ausgeführt, die in die Trennebene 54 des aus zwei Teilen bestehenden Tray 40 eingelegt werden.

Mit der modifizierten Anordnung nach Fig. 8 können in einer weiteren Ausgestaltung der Erfindung, wie in Fig. 9 dargestellt, auch Kompartimente aus zwei Kompartimentenströmen 3a und 3b in einem gemeinsamen Kompartimentenstrom 3 vereinigt werden. Zu diesem Zweck wird wie in Fig. 9 dargestellt, der Aufsetzsonde 51 und der Aufsetzsonde 53 jeweils ein Kompartimentenstrom 3a und 3b zugeführt. Die Vereinigung der Kompartimentenströme 3a und 3b erfolgt an der Stirnfläche 5 des PTFE-Schlauches 7 der Aufsetzsonde 52. Zur Synchronisation der beiden Kompartimentenströme der Aufsetzsonden 51 und 53 werden Detektoren 60a und 60b eingesetzt, welche in den Verbindungskanälen 27 angeordnet und zur Ermittlung der Phasengrenzen vorgesehen sind. Je nach Synchronisation der Steuerung erfolgt dabei eine Verschachtelung, d.h. abwechselnd wird ein Kompartiment aus dem Kompartimentenstrom 3a und ein Kompartiment aus dem Kompartimentenstrom 3b der Aufsetzsonde 52 zugeführt, oder ein Vereinigen, d. h. jeweils ein Kompartiment des Kompartimentenstroms 3a wird mit einem Kompartiment aus dem Kompartimentenstrom 3b zu einem großen Kompartiment zusammengefügt und durch die Aufsetzsonde 52 aufgenommen. Das heißt, dass zum Selektieren oder Aufteilen eines Kompartimentenstroms 3 in mehrere Kompartimentenströme 3a, 3b die Well Selktionszuführung 47 entweder über mindestens zwei Ausgänge, aus denen abwechselnd einer der Kompartimentenströme 3a, 3b mit der Flussrate Φ4, Φ8 abgesaugt wird und mit Hilfe der Detektoren 60, 60a, 60b zur Erkennung von Phasengrenzen im Kompartimentenstrom 3 Pumpen 71, 72 zum Absaugen der Kompartimentenströme 3a, 3b gesteuert werden, verfügt oder dass zum Vereinigen von Kompartimenten aus zwei Kompartimentenströmen 3a, 3b diese den Enden der ineinander verschachtelter Fluidleiter 1, 2 der Aufsetzsondesonde 52 zugeführt werden, wobei durch den inneren Fluidleiter 1 der vereinigte Kompartimentenstrom abgesaugt wird und durch den äußeren Fluidleiter 2 Transportflüssigkeit 16 zugeführt wird und mit Detektoren 60 zur Erkennung von Phasengrenzen in den Kompartimentenstrom 3a, 3b Pumpen zum Antrieb der Kompartimentenströme 3a, 3b gesteuert werden.

Die Anordnung nach Fig. 8 kann auch zum Setzen von Markern an bestimmten Positionen des Kompartimentenstroms verwendet werden. Auch bei diesen Anwendungen wird vorausgesetzt, dass die Flussraten der Kompartimentenströme 3a und 3b, mit denen die Aufsetzsonden 51 und 53 beaufschlagt werden, exakt gesteuert werden. Es können natürlich auch beispielsweise die Kompartimentenströme 3a und 3b ohne Hilfe von Aufsetzsonden direkt in das Well 82 zum weiteren Prozessieren geleitet werden.

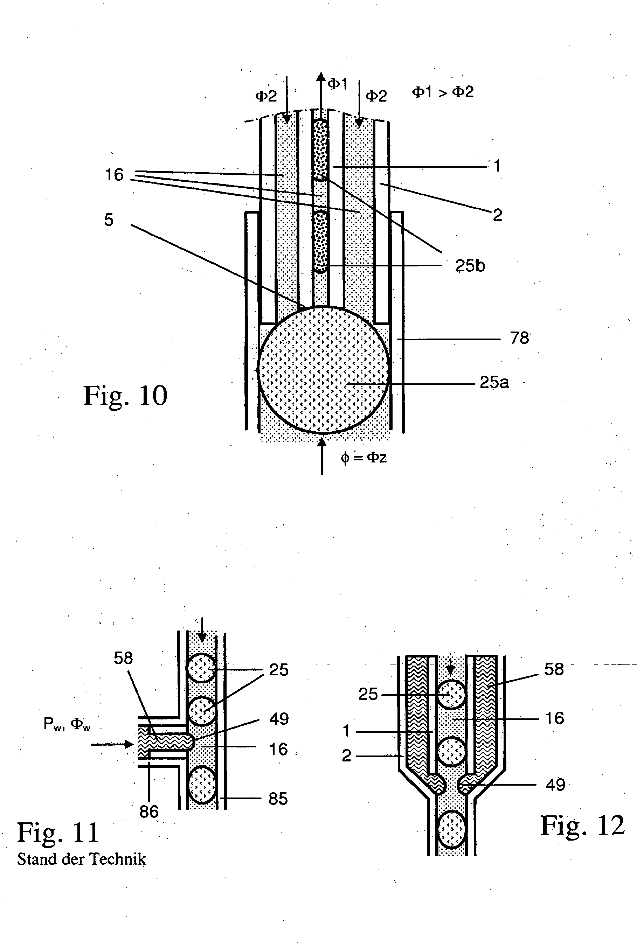

Eine weitere Ausführung der Erfindung betrifft das Splitten eines großen Kompartimentes in viele kleinere. Das Wirkprinzip ist in Fig. 10 dargestellt. Die ineinander verschachtelten Fluidleiter 1 und 2 tauchen zu diesem Zweck in einen Zuführkanal 78, dessen Innendurchmesser etwa dem Außendurchmesser des Fluidleiters 2 bzw. der Kanüle 8 entspricht. Berührt das große Kompartiment 25a die Stirnfläche 5 des inneren Fluidleiters 1, setzt bei $\Phi1 > \Phi2$ ein Kompartimentiervorgang ein, der das große Kompartiment 25a in viele kleine Kompartimente 25b zerlegt. Die Flussrate Fz im Zuführkanal 78 wird dabei durch $\Phi1$ und $\Phi2$ bestimmt. Es gilt:

$$\Phi1 = \Phi2 + Fz$$

Der Kompartimentenstrom 3, der aus den kleinen Kompartimenten 25b besteht, kann dann in einer Anordnung, wie in Fig. 8 und 9 dargestellt, auf verschiedene Stationen aufgeteilt werden.

In einer anderen Betriebsart wird auf den Kompartimentenstrom im Zuführkanal 78 ein Fluss aufgeprägt, während Fluss f1, f2 = nn ist. In diesem Fall teilt sich das Kompartiment an der Stirnfläche 5 in ein Kompartiment, dass den inneren Fluidleiter 1 verlässt und in ein toroidförmiges Kompartiment, dass den Fluidleiter 2 verlässt. Nachdem dieses toroidförmige Kompartiment die Durchführung 6 verlässt, nimmt es dann wieder eine Kugelform an.

In einer weiteren Ausgestaltung der Erfindung wird die Aufsetzsonde in Verbindung mit einem speziellen Well beispielsweise zur Zudosierung von Effektoren eingesetzt. Solche chemisch, physikalisch und biologisch wirksamen Effektoren wie beispielsweise Antibiotika, werden zum einen für die Veränderung der Mikrosphäre in den Kompartimenten, die das Probenmaterial umgibt, oder für eine Veränderung direkt am Probenmaterial, verwendet. Zum anderen ist es aber durch Zugabe von Effektoren möglich, die Grenzfläche zwischen dem Kompartiment und dem umgebenden Fluid so zu beeinflussen, dass beispielsweise eine Verkapselung des Kompartimentes in eine stabile Hülle erfolgt.

Die Funktionsweise des Zudosierens nach Stand der Technik wird in Fig. 11 gezeigt. In einem Fluidleiter 85 wird ein Strom von Kompartimenten 25 bewegt. Senkrecht zum Fluidleiter 85 ist ein weiterer Fluidleiter als Zudosierkanal 86 angeordnet und mit Fluidleiter 85 fluidisch verbunden. In diesen weiteren Fluidleiter 86 wird ein Fluid 58 in Richtung Fluidleiter 1 gedrückt, wobei das Fluid 58 bezüglich der Mischbarkeit mit den Kompartimenten 25 vergleichbare Eigenschaften besitzt und einen Meniskus 49 in das Transportfluid 16 ausbildet. Ein im Fluidleiter 85 transportiertes Kompartiment 25 berührt den Meniskus 49, wobei es zu einem Vermischen mit dem Kompartiment 25 und zur Mitnahme eines Teils des Fluids 58 kommt. Durch die Variation der geometrischen und prozesstechnischen Parameter ist das Volumen des durch das Kompartiment 25 aufgenommenen Fluids 58 vorwählbar. Der Prozess des Zudosierens kann dabei durch einen Fluss- oder Druckimpulse $F_w$ bzw. $P_w$ unterstützt werden.

Die Anwendung des Prinzips auf röhrenförmige, ineinander verschachtelte Fluidleiter, wie sie der erfindungsgemäßen Anordnung entspricht, wird in Fig. 12 dargestellt. Die Kontaktfläche des Meniskus 49 des Fluids 58 mit dem Kompartiment 25 kann dabei kreisrund sein. Dadurch werden gegenüber einem punktförmigen Kontakt Vorteile erreicht, wenn zum Beispiel eine Verkapselung des Kompartimentes durch Umhüllung des Kompartimentes mit einem geeigneten Effektor 13 begünstigt werden soll.

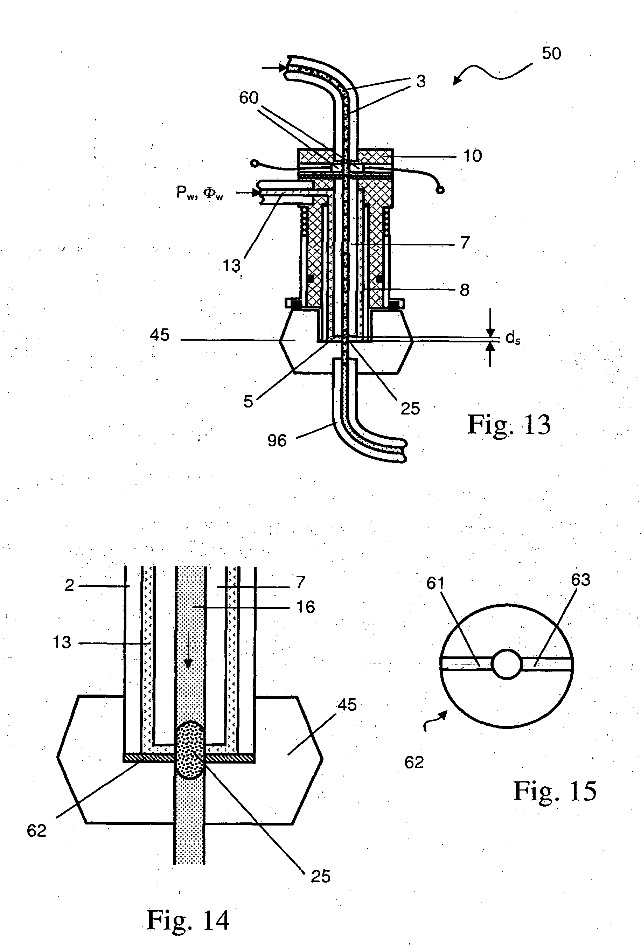

Die technische Ausführung der Zudosierung in einer Aufsetzsonde 50 nach dem Prinzip in Fig. 12 wird in Fig. 13 verdeutlicht. Durch den äußeren Fluidleiter 2, der sich röhrenförmig zwischen dem PTFE-Schlauch 7 und der Kanüle 8 ausbildet, wird beispielsweise ein Effektor 13 transportiert. Die Kanüle 8 ist zu diesem Zweck gegenüber der Stirnfläche 5 des inneren Fluidleiters 7 um einen Betrag $d_s$, beispielsweise 0,1 mm länger, sodass beim Aufsetzen der Aufsetzsonde 50 in das für eine Zudosierung vorgesehene Well 45 ein Spalt mit der Breite $d_s$ = 0,1 mm entsteht. Dabei setzt die Kanüle 8 flüssigkeitsdicht auf dem Boden des Wells 45 auf, wobei dass Well 45 über einen Ablauf 96 zur Abführung des zudosierten Kompartimentstroms 3 verfügt. Wird nun beispielsweise der Effektor 13 durch den Fluidleiter 2 gefördert, wird dieser durch den Spalt $d_s$ in das Kompartiment 25 aufgenommen. Der Kompartimentenstrom wird durch das Well 45 und einem Verbindungsschlauch 96 abgeleitet.

In dem Fall, dass die Zugabe eines Effektors 13 durch einen Fluss- oder Druckimpuls $F_w$ bzw. $P_w$ unterstützt werden soll, ist eine exakte Synchronisation zwischen dem Zeitpunkt des Impulses und der Passage des Kompartimentes an der Stirnfläche 5 des inneren Fluidleiters 1 notwendig. In einer vorteilhaften Ausführung der Erfindung wird die exakte Position des Kompartimentes an der Stirnfläche 5 durch eine Messung der Änderung der Impedanz zwischen einer Elektrodenanordnung ermittelt. Dazu wird, wie in Fig. 14 und 15 gezeigt ist, der Boden des Wells mit einer Elektrodenplatte 62 mit zwei Elektroden 61 und 63 versehen. Die messbare Impedanzänderung zwischen den Elektroden 61 und 63 beim Durchlauf eines Kompartimentes 25 beruht auf den unterschiedlichen dielektrischen Eigenschaften des Transportfluids 16 und eines Kompartimentes 25 und dient der Erkennung von Phasengrenzen im Kompartimentenstrom 3.

Eine andere Variante zur Synchronisation zwischen den Kompartimentenstrom und der Zudosierung eines Effektors ist ebenfalls in Fig. 13 angedeutet. Dabei werden optische Detektoren 60 in der Art einer Lichtschranke im Sondenanschluss 10 angeordnet, die den Durchgang eines Kompartiments 25 registrieren und somit zur Erkennung der Phasengrenzen im Kompartimentenstrom 3 geeignet sind.

Die für die Zudosierung vorgenommene Modifikation der Aufsetzsonde 50 stellt im übrigen keine Funktionseinschränkung für das Kompartimentieren dar, sodass ein Typ von Aufsetzsonde mit allen beschrieben Erfindungsmerkmalen für die verschiedenen Aufgaben des Kompartimentierens, Dekompartimentierens, Speicherns, Spülens, Zudosierens, Vereinigen, Zudosierung von Effektoren, Zusammenführen/Prozessierens und Verzweigen/Selektieren von Kompartimentenströmen sowie Detektierens/Analysierens eingesetzt werden kann. Lediglich die Betriebsart der Aufsetzsonde 50 hinsichtlich der Art der zugeführten Fluide und der Größe der aufgeprägten Flussraten bzw. Drücke entscheidet in Wechselwirkung mit einem speziellen Well über die jeweilige Aufgabe.

Auf der Grundlage dieser mit allen erfindungsgemäßen Merkmalen ausgestatteten Aufsetzsonde lässt sich ein flexibel konfigurierbares Laborsystem aufbauen. Ein solches Laborsystem besteht aus einer oder mehreren Aufsetzsonden 50 identischer Bauart, einem Tray 40, das die unterschiedlichen Wells aufnimmt, Pumpen zur Aufprägung von Flüssen verschiedener Fluide, Ventile, Vorratsbehälter für verschiedene Fluide, Speicher und eine elektronische Systemsteuerung in beliebiger Kombination.

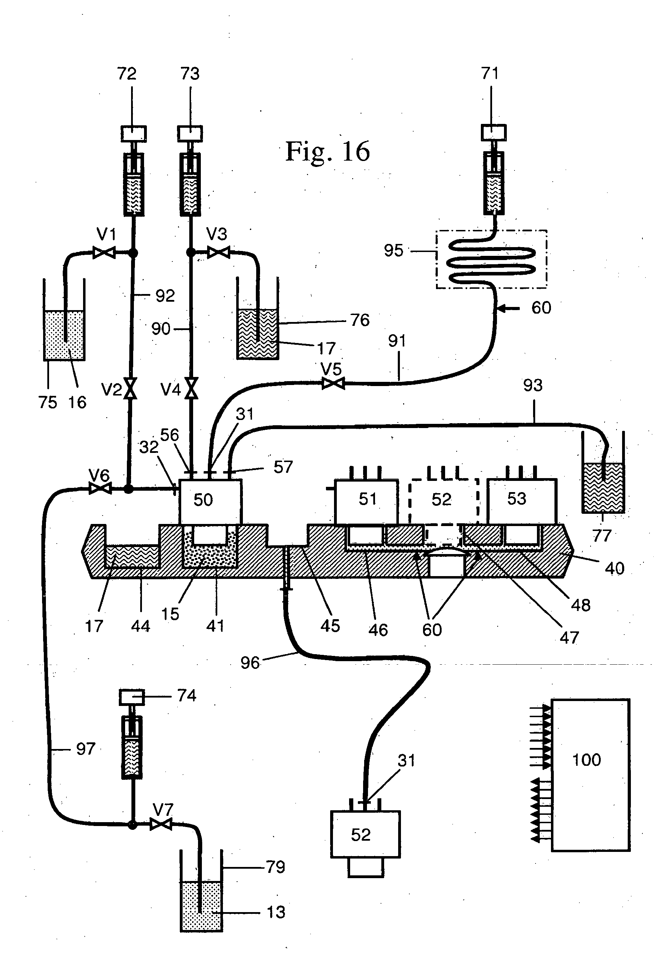

In Fig. 16 wird ein Ausführungsbeispiel für ein einfaches komplettes Laborsystem mit insgesamt vier baugleichen Aufsetzsonden 50, 51, 52, 53 dargestellt, das beispielsweise für die Funktionen Kompartimentieren, Speichern, Zudosieren von Effektoren, Spülen und Selektieren von Kompartimenten ausgelegt ist. Jede der Aufsetzsonden besitzt einen Schlauchanschluss 31 für den inneren Fluidleiter 1, einen Schlauchanschluss 32 für den äußeren Fluidleiter 2 sowie zwei weitere Anschlüsse 56, 57 für das Spülfluid 17 bzw. für den Effektor 13. In Fig. 16 übernimmt die Aufsetzsonde 50 zunächst die Aufgabe des Kompartimentierens. Zu diesem Zweck ist der Anschluss 32 für den äußeren Fluidleiter 2 über den Verbindungsschlauch 92 mit der Spritzenpumpe 72 verbunden. Der Verbindungsschlauch 91 verbindet den Schlauchanschluss 31 für den inneren Fluidleiter 1 mit dem Speicherschlauch 95 und der Spritzenpumpe 71.

Vor dem eigentlichen Kompartimentiervorgang, wird zunächst die Spritzenpumpe 72 bei geschlossenen Ventil V2 und geöffneten Ventil V1 1 mit Transportfluid 16 aus dem Vorratsbehälter 75 gefüllt. Die Spritzenpumpe 71 ist dagegen entleert. Im Speicherschlauch 95 bzw. im Verbindungsschlauch 91 befindet sich Transportfluid 16.

Während des Kompartimentierens sind die Ventile V1, V4, V6 geschlossen und die Ventile V2, V5 offen. Die Spritzenpumpe 72 prägt einen Fluss des Transportfluids 16 mit der Flussrate Φ2 auf den äußeren Fluidleiter 2 auf. Gleichzeitig saugt die Spritzenpumpe 71 den erzeugten Kompartimentenstrom 3 mit einer Flussrate Φ1 aus dem inneren Fluidleiter 1 ab. Der Detektor 60 zur Erkennung der Phasengrenzen im Kompartimentenstrom 3 zeigt den Einlauf des Kompartimentenstroms 3 in den Schlauchspeicher 95 an. Der Vorgang wird maximal so lange fortgesetzt, bis der Schlauchspeicher 95 mit dem Kompartimentenstrom 3 gefüllt ist.

Anschließend soll beispielsweise ein Spülvorgang vorgenommen werden. Zu diesem Zweck wird die Aufsetzsonde 50 manuell oder durch ein hier nicht dargestelltes automatisches Handhabegerät auf das für eine Spülung vorgesehene Well 44 umgesetzt. Die vorher aus dem Vorratsbehälter 76 bei geöffneten Ventil V3 gefüllte Spritzenpumpe 73 drückt Spülfluid 17 über den Verbindungsschlauch 90 und den Schlauchanschluss 56 durch die Spülkanäle 33, 34 sowie die durch die Aufsetzsonde 50 und dem Well 44 gebildete Kammer 84 (vergleiche Fig. 5 und Fig. 6). Während dieses Vorganges sind die Ventile V2, V3, V5, V6 geschlossen. Das überschüssige Spülfluid 17 gelangt über den Schlauchanschluss 57 und den Schlauch 93 in einen Abfall - Behälter 77.

Soll der im Speicherschlauch 95 befindliche Kompartimentenstrom 3 mit Effektoren 13 behandelt werden, wird zunächst die Aufsetzsonde 50 auf das für eine Zudosierung vorgesehene Well 45 manuell oder mit einem elektronisch gesteuerten Handhabungsgerät umgesetzt. Die Spritzenpumpe 74 wird mit Effektor 13 aus dem Vorratsbehälter 79 bei geöffneten Ventil V7 und geschlossenen Ventil V6 gefüllt. Anschließend werden die Ventile V2, V4, V7 geschlossen und die Zudosierung erfolgt in der im Zusammenhang mit Fig. 13 bis 15 beschriebenen Weise mit Hilfe der Spritzenpumpe 71 zur Entleerung des Kompartimentenstroms 3 aus dem Speicherschlauch 95 und mit Hilfe koordiniert eingeleiteter Druck- oder Flussimpulsen $P_W$, $F_W$ durch die Spritzenpumpe 74 beim Kompartimentendurchlauf an der Stirnfläche 5 der Aufsetzsonde 50.

Der mit Effektoren 13 behandelte Kompartimentenstrom 3 wird dem Well 45 durch den Verbindungsschlauch 96 entnommen und dem Anschluss 31 für den Fluidleiter 1 der Aufsetzsonde 52 zugeführt. Die Aufsetzsonde 52 kann auf das für die Selektionszuführung vorgesehene Well 47 aufgesetzt werden. Die einzelnen Kompartimente 25 werden, wie im Zusammenhang mit Fig. 8 erläutert, beispielsweise einer optischen Beobachtung unterzogen und werden, je nach Selektionskriterium durch die Aufsetzsonden 51 oder 53 entnommen. Die entsprechenden Schlauchverbindungen und weiteren Spritzenpumpen, die für den Betrieb der Aufsetzsonden 51, 52 und 53 notwendig sind, wurden der Übersichtlichkeit wegen nicht dargestellt.

Die Systemsteuerung 100 übernimmt die Ablaufsteuerung der Ventile und Spritzenpumpen sowie die Auswertung der Detektoren für den Kompartimentendurchlauf und sonstige Sensoren. Unter Einbeziehung der im Zusammenhang mit dem Beispiel nach Fig. 16 nicht genannten Funktionen wie Prozessieren, Dekompartimentieren, Aufsplitten und Vereinigen von Kompartimenten sowie Zusammenführen und Verzweigen von Kompartimentenströmen ergeben sich vielfältige weitere Möglichkeiten zur Gestaltung eines komplexen Laborsystems in Anpassung an die jeweiligen Aufgaben.

Die Stabilität des Kompartimentiervorganges hängt wesentlich davon ab, wie es gelingt, die Fluidleiter 1 und 2 einen konstanten Fluss $\Phi 1$ und $\Phi 2$ unabhängig von variablen Strömungswiderständen aufzuprägen. Für diesen Zweck sind zum Beispiel Spritzenpumpen geeignet, bei dem ein Kolben über ein Schraubgetriebe durch einen Schrittmotor angetrieben und in eine lineare Bewegung versetzt wird. Ein Schritt des Schrittmotors entspricht dabei einem bestimmten Volumen.

Für die Erzeugung der Flüsse $\Phi 1$ und $\Phi 2$ sind entweder zwei Spritzenpumpen mit vorwählbarer Schrittmotordrehzahl oder eine Spritzenpumpe mit zwei gegenläufigen Spritzen unterschiedlicher Volumina, aber einer gemeinsamen vorwählbaren Schrittmotordrehzahl vorteilhaft. Die Anwendung zweier Spritzenpumpen bietet den Vorteil, dass die Schrittmotoren beider Spritzenpumpen mittels einer übergeordneten Steuersoftware unabhängig voneinander angesteuert werden können und dadurch das Kompartimentierverhalten beeinflusst werden kann. Die Größe der Kompartimente und deren Abstand können hierdurch oder durch Variation des Start-Stop-Verhaltens gezielt vorgewählt werden.

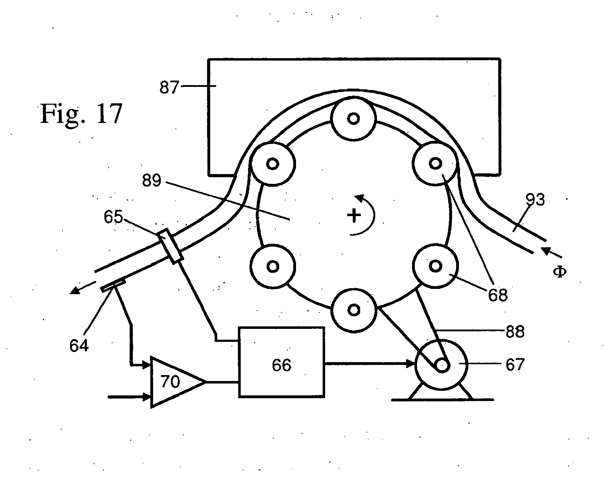

Anstelle der in der Fig. 16 dargestellten Spritzenpumpen 71 bis 73 können auch beispielsweise wie in Fig. 17 dargestellte geregelte Schlauchpumpen verwendet werden. Damit werden weniger Ventile benötigt. Die für diesen Einsatzfall ungünstige Kennlinie der Schlauchpumpen (vom Gegendruck stark abhängige Förderleistung) kann durch Einbinden in einen Regelkreis nach Fig. 17 angepasst werden kann. Sensor 64 in diesem Regelkreis ist ein Flussmesser z.B. auf bolometrischer Basis bekannter Bauart. Dem Fluidstrom F wird durch ein Heizelement 65 eine lokale Erhitzung aufgeprägt. Die Laufzeit der Temperaturerhöhung bis zum Thermosensor 64 wird gemessen und mit einem Sollwert durch den Komparator 70 verglichen. Die ermittelte Abweichung führt nach Signalverarbeitung in einem Mikroprozessor 66 wiederum zu einer Änderung der Winkelgeschwindigkeit des Antriebsmotors 67, der den Rollenkäfig 89 der Schlauchrollen 68 über einen Antriebsriemen 88 antreibt. Somit sind zur Aufprägung von Flüssen in den inneren Fluidleiter 1 und den äußeren Fluidleiter 2 Messeinrichtungen zur Ermittlung des Ist-Flusses vorhanden, wobei dieser Ist-Fluss mit einem Sollfluss verglichen werden kann und die ermittelte Differenz eine Nachregelung der Pumpleistung bewirkt.

Bezugszeichenliste

| | |
|---|---|
| 1 | Innerer Fluidleiter |
| 2 | Äußerer Fluidleiter |
| 3 | Kompartimentenstrom |
| 3a | Kompartimentenstrom |
| 3b | Kompartimentenstrom |
| 4 | Well |
| 5 | Stirnfläche |
| 6 | Durchführung |
| 7 | PTFE-Schlauch |
| 8 | Kanüle |
| 9 | Sondenkörper |
| 10 | Sondenanschluss |
| 11 | unpolares Fluid |
| 12 | polares Fluid |
| 13 | Effektor |
| 14 | |
| 15 | Probenfluid |
| 16 | Transportfluid |
| 17 | Spülfluid |
| 18 | selektiertes Kompartiment |
| 19 | Auswertung |
| 20 | Probenmeniskus |
| 21 | Position 21 des Probenmeniskus |
| 22 | Position 22 des Probenmeniskus |
| 23 | Position 23 des Probenmeniskus |
| 24 | |
| 25 | Kompartiment |
| 26 | Bedeckungsfleck |
| 27 | Verbindungskanal |
| 28 | Einlaufschräge |
| 29 | Belüftungskanal |
| 30 | Filter |
| 31 | Schlauchanschluss Kompartimentenstrom |
| 32 | Schlauchanschluss Transportfluid |
| 33 | Spülkanal Zulauf |
| 34 | Spülkanal Ablauf |
| 35 | Hülse |
| 36 | Dichtplatte |
| 37 | Führungsdichtung |
| 38 | Aufsetzdichtung |
| 39 | Spiralfeder |
| 40 | Tray |

| | |
|---|---|
| 41 | Well Probenfluid A |
| 42 | Well Probenfluid B |
| 43 | Well Probenfluid C |
| 44 | Well Spülfluid |
| 45 | Well Zudosierung |
| 46 | Well Sortenabführung A |
| 47 | Well Selektionszuführung |
| 48 | Well Sortenabführung B |
| 49 | Effektor-Meniskus |
| 50 | Aufsetzsonde |
| 51 | Aufsetzsonde A |
| 52 | Aufsetzsonde B |
| 53 | Aufsetzsonde C |
| 54 | Trennebene |
| 55 | Anschluss für peripheren Fluidleiter |
| 56 | Anschluss für Spülfluid Zulauf |
| 57 | Anschluss für Spülfluid Ablauf |
| 58 | Fluid mischbar mit Kompartiment 25 |
| 59 | Koppelschlauch |
| 60 | Detektor Kompartiment |
| 60a | Detektor |
| 60b | Detektor |
| 61 | Gegenelektrode |
| 62 | Elektrodenplatte |
| 63 | Messelektrode |
| 64 | Thermosensor |
| 65 | Heizelement |
| 66 | Mikroprozessor |
| 67 | Stellmotor |
| 68 | Schlauchrollen |
| 69 | Optik |
| 70 | Komparator |
| 71 | Spritzenpumpe A |
| 72 | Spritzenpumpe B |
| 73 | Spritzenpumpe C |
| 74 | Spritzenpumpe D |
| 75 | Behälter für Transportfluid |
| 76 | Behälter für Spülfluid |
| 77 | Abfall - Behälter |
| 78 | Zuführkanal |
| 79 | Vorratsbehälter für Effektor |
| 80 | |
| 81 | Well Sortenzuführung A |
| 82 | Well Zusammenführung |
| 83 | Well Sortenzuführung B |
| 84 | Kammer |
| 85 | Fluidleiter allgemein |
| 86 | Zudosierkanal |
| 87 | Gegenhalter |
| 88 | Antriebsriemen |
| 89 | Rollenkäfig |
| 90 | Verbindungsschlauch |
| 91 | Verbindungsschlauch |
| 92 | Verbindungsschlauch |
| 93 | Verbindungsschlauch |
| 94 | |
| 95 | Speicherschlauch |
| 96 | Verbindungsschlauch |

| | |
|---|---|
| 97 | Verbindungsschlauch |
| 98 | |
| 99 | |
| 100 | Systemsteuerung |

Φ1 bis Φ8    Fluss / Flussrate

**Patentansprüche**

1. Anordnung zum Erzeugen, Manipulieren und Analysieren von Kompartimenten, mit mindestens zwei Fluidleitern (1, 2) zum Führen voneinander getrennter Fluidpfade, **dadurch gekennzeichnet, dass** zwei Fluidleiter (1, 2) ineinander geschachtelt angeordnet sind, wobei ein Ende eines ersten Fluidleiters (2) mit einer steuerbaren Flussquelle für die Ausprägung eines Flusses eines Transportfluids (16) verbunden ist, ein Ende eines zweiten Fluidleiters (1) mit einer weiteren steuerbaren Flussquelle für die Aufprägung eines Flusses eines Kompartimentenstroms (3) verbunden ist und beide Fluidleiter (1, 2) mit ihrem jeweils anderen Ende in ein Fluidreservoir (4) eingetaucht sind, in dem sich ein weiteres Fluid (15), befindet, welches mit dem Transportfluid (16) nicht mischbar ist.

2. Anordnung nach Anspruch 1, **dadurch gekennzeichnet, dass** der innere Fluidleiter (1) an seinem in das Fluidreservoir (4) eintauchenden Ende mit einer Stirnfläche (5) ausgestattet ist, die vollständig oder partiell nicht benetzend für das im Fluidreservoir (4) befindliche Fluid (15) ist.

3. Anordnung nach Anspruch 1 oder 2, **dadurch gekennzeichnet, dass** die innere Oberfläche des Fluidleiters (1) für den Kompartimentenstrom (3) vollständig oder partiell nicht benetzend für das im Fluidreservoir befindliche Fluid (15) ist.

4. Anordnung nach einem der vorhergehenden Ansprüche, **dadurch gekennzeichnet, dass** die Fluidleiter (1, 2) in einer Aufsetzsonde (50) angeordnet sind, die einen Sondenkörper (9) mit separaten Schlauchanschlüssen (31, 32) für den Kompartimentenstrom (3) und für das Transportfluid (16) enthält.

5. Anordnung nach einem der vorhergehenden Ansprüche, **dadurch gekennzeichnet, dass** das Fluidreservoir (4) als Well ausgeführt ist, und an der Aufsetzsonde (50) Dichtmittel (37, 38) angeordnet sind, mit denen nach dem Aufsetzen der Aufsetzsonde (50) auf das Well (4) das Fluid (15) im Well (4) gegenüber der Umgebung gas- und/oder flüssigkeitsdicht abgeschlossen wird.

6. Anordnung nach einem der Ansprüche 4 oder 5, **da-**

**durch gekennzeichnet, dass** im Sondenkörper (9) Spülkanäle (33), (34) zum Durchleiten eines Spülfluids (17) und/oder ein Belüftungskanal (29) angeordnet ist, der mit einem Filter (30) steril verschlossen ist, angeordnet sind.

7. Anordnung nach einem der Ansprüche 4 bis 6, **dadurch gekennzeichnet, dass** in der Aufsetzsonde (50) Detektoren (60) zur Erkennung der Phasengrenzen im Kompartimentenstrom (3) angeordnet sind.

8. Anordnung nach Anspruch 7, **dadurch gekennzeichnet, dass** das Fluidreservoir (4) mehrere Wells enthält, wobei mindestens zwei Wells durch Verbindungskanäle (27) verbunden sind, in denen Detektoren (60, 60a, 60b) für die Ermittlung von Phasengrenzen angeordnet sind.

9. Anordnung nach einen der Ansprüche 4 bis 8, **dadurch gekennzeichnet, dass** der äußere Fluidleiter (2) gegenüber dem inneren Fluidleiter (1) um einen Betrag $d_s$ übersteht, der äußere Fluidleiter (2) flüssigkeitsdicht auf den Boden eines Wells (45) aufsetzt, das Well (45) über einen Ablauf (96) zur Abführung des zudosierten Kompartimentenstroms verfügt und dass Detektoren (60,62) zur Erkennung von Phasengrenzen im Kompartimentenstrom (3) vorhanden sind.

10. Verfahren zum Erzeugen, Manipulation und Analysieren von Kompartimenten, **dadurch gekennzeichnet, dass** mittels zweier, ineinander verschachtelter Flüssigkeitsleiter (1, 2), die jeweils mit einem Ende in ein Fluidreservoir (4) mit Fluid A eintauchen, dem inneren Fluidleiter (1) der Fluss ($\Phi$1) eines Fluids B aufgeprägt wird, und dem äußeren Fluidleiter (2) ein Fluss ($\Phi$2) eines Fluids C aufgeprägt wird, wobei die Fluids A und B nicht mischbar sind.

11. Verfahren nach Anspruch 10, **dadurch gekennzeichnet, dass** zur Erzeugung von Kompartimenten das Fluidreservoir (4) mit Probenfluid (15) gefüllt wird, der äußere Fluidleiter (2) mit einem Fluss ($\Phi$2) eines Transportfluids (16) beaufschlagt wird und durch den inneren Fluidleiter (1) ein Kompartimentenstrom (3) mit einem Fluss ($\Phi$1) abgesaugt wird, wobei der Fluss ($\Phi$1) größer als der Fluss ($\Phi$2) ist.

12. Verfahren nach Anspruch 10, **dadurch gekennzeichnet, dass** zum Dekompartimentieren des Kompartimentenstroms (3) durch den inneren Fluidleiter (1) eine Flussrate ($\Phi$1) in das Fluidreservoir (4) fließt, das ein mit den Kompartimenten mischbares Fluid enthält, und durch den äußeren Fluidleiter (2) Transportflüssigkeit (16) mit der Flussrate ($\Phi$2) abgesaugt wird.

13. Verfahren nach Anspruch 10, **dadurch gekennzeichnet, dass** zum Selektieren oder Aufteilen eines Kompartimentenstroms (3) in mehrere Kompartimentenströme (3a, 3b) das Fluidreservoir (47) über mindestens zwei Ausgänge verfügt, aus denen abwechselnd einer der Kompartimentenströme (3a. 3b) mit der Flussrate ($\Phi$4, $\Phi$8) abgesaugt wird und mit Hilfe von Detektoren (60) zur Erkennung von Phasengrenzen im Kompartimentenstrom (3) Pumpen (71, 72) zum Absaugen der Kompartimentenströme (3a, 3b) gesteuert werden.

14. Verfahren nach Anspruch 13, **dadurch gekennzeichnet, dass** für die Ermittlung eines Selektionskriteriums zur Aufteilung des Kompartimentenstroms (3) das Fluidreservoir (47) eine Analysevorrichtung (69, 19) enthält, die zur Ermittlung des Zustandes der einzelnen Kompartimente verwendet wird.

15. Verfahren nach Anspruch 10, **dadurch gekennzeichnet, dass** zum Vereinigen von Kompartimenten aus zwei Kompartimentenströmen (3a, 3b) diese den Enden der ineinander verschachtelter Fluidleiter (1, 2) der Aufsetzsondesonde (52) zugeführt werden, wobei durch den inneren Fluidleiter (1) der vereinigte Kompartimentenstrom abgesaugt wird und durch den äußeren Fluidleiter (2) Transportflüssigkeit (16) zugeführt wird und mit Detektoren (60) zur Erkennung von Phasengrenzen in den Kompartimentenstrom (3a, 3b) Pumpen zum Antrieb der Kompartimentenströme (3a, 3b) gesteuert werden.

16. Verfahren nach Anspruch 10, **dadurch gekennzeichnet, dass** zum Aufsplitten eines Kompartimente in mehrere kleine Kompartimente dieses durch einen Zuführkanal (78), dessen Innendurchmesser dem Außendurchmesser des Fluidleiters (2) entspricht, direkt den Enden der ineinander verschachtelter Fluidleiter (1, 2) zugeführt wird, bei denen durch den inneren Fluidleiter (1) die aufgesplitteten Kompartimente abgesaugt werden und durch den äußeren Fluidleiter Transportflüssigkeit zugeführt wird.

17. Verfahren nach Anspruch 10, **dadurch gekennzeichnet, dass** zum Zudosieren eines Effektors zum Kompartiment dem äußeren Fluidleiter (2) ein Effektor (13) zugeführt wird, und dass Detektoren (60) zur Erkennung von Phasengrenzen im Kompartimentenstrom (3), bestehend aus Kompartimenten (25) und Transportflüssigkeiten (16), verwendet werden, um eine Pumpe (71), die mit einem Schlauchanschluss (31) verbunden ist, zu steuern.

18. Verfahren nach einem der Ansprüche 10 bis 17, **dadurch gekennzeichnet, dass** zur Ausprägung von Flüssen in den inneren Fluidleiter (1) und dem äußeren Fluidleiter (2) Messeinrichtungen zur Ermittlung des Ist-Flusses vorhanden sind, dieser Ist-Fluss mit einem Sollfluss verglichen wird und die ermittelte Differenz eine Nachregelung der Pumpleistung bewirkt.

## Claims

1. Assembly for generating, manipulating and analysing compartments, comprising at least two fluid conductors (1, 2) for guiding fluid paths that are separate from one another, **characterized in that** two fluid conductors (1, 2) are arranged nested one inside the other, one end of a first fluid conductor (2) being connected to a controllable flow source for imparting a flow of a transport fluid (16), one end of a second fluid conductor (1) being connected to a further controllable fluid source for imparting a flow of a compartment stream (3) and both fluid conductors (1, 2) being submerged with their other end, respectively, in a fluid reservoir (4), in which there is a further fluid (15), which is immiscible with the transport fluid (16).

2. Assembly according to Claim 1, **characterized in that** the inner fluid conductor (1) is provided at its end that is submerged in the fluid reservoir (4) with an end face (5), which is completely or partially non-wetting for the fluid (15) that is in the fluid reservoir (4).

3. Assembly according to Claim 1 or 2, **characterized in that** the inner surface of the fluid conductor (1) for the compartment stream (3) is completely or partially non-wetting for the fluid (15) that is in the fluid reservoir.

4. Assembly according to one of the preceding claims, **characterized in that** the fluid conductors (1, 2) are arranged in a surface-mounted probe (50), which includes a probe body (9) with separate tube connections (31, 32) for the compartment stream (3) and for the transport fluid (16).

5. Assembly according to one of the preceding claims, **characterized in that** the fluid reservoir (4) is configured as a well and sealing means (37, 38), with which the fluid (15) in the well (4) is closed off from the surroundings in a gas- and/or liquid-tight manner after the surface-mounted probe (50) has been mounted on the well (4), are arranged on the surface-mounted probe (50).

6. Assembly according to either of Claims 4 and 5, **characterized in that** flushing channels (33), (34) for passing a flushing fluid (17) through and/or an air-admitting channel (29), which is sealed in a sterile manner with a filter (30), are arranged in the probe body (9).

7. Assembly according to one of Claims 4 to 6, **characterized in that** detectors (60) for detecting the phase boundaries in the compartment stream (3) are arranged in the surface-mounted probe (50).

8. Assembly according to Claim 7, **characterized in that** the fluid reservoir (4) contains multiple wells, at least two wells being connected by connecting channels (27), in which detectors (60, 60a, 60b) for the determination of phase boundaries are arranged.

9. Assembly according to one of Claims 4 to 8, **characterized in that** the outer fluid conductor (2) projects beyond the inner fluid conductor (1) by an amount $d_s$, the outer fluid conductor (2) is mounted in a fluid-tight manner on the bottom of a well (45), the well (45) has an outlet (96) for the removal of the metered-in compartment stream and **in that** there are detectors (60, 62) for detecting phase boundaries in the compartment stream (3).

10. Method for generating, manipulating and analysing compartments, **characterized in that**, by means of two liquid conductors (1, 2) that are nested one inside the other and are respectively submerged with one end in a fluid reservoir (4) with fluid A, the flow ($\Phi1$) of a fluid B is imparted to the inner fluid conductor (1) and a flow ($\Phi2$) of a fluid C is imparted to the outer fluid conductor (2), the fluids A and B being immiscible.

11. Method according to Claim 10, **characterized in that**, for generating compartments, the fluid reservoir (4) is filled with sample fluid (15), the outer fluid conductor (2) is subjected to a flow ($\Phi2$) of a transport fluid (16) and a compartment stream (3) is sucked away through the inner fluid conductor (1) with a flow ($\Phi1$), the flow ($\Phi1$) being greater than the flow ($\Phi2$).

12. Method according to Claim 10, **characterized in that**, for decompartmentalizing the compartment stream (3), a flow rate ($\Phi1$) flows through the inner fluid conductor (1) into the fluid reservoir (4), which contains a fluid that is miscible with the compartments, and transport fluid (16) is sucked away through the outer fluid conductor (2) with the flow rate ($\Phi2$).

13. Method according to Claim 10, **characterized in that**, for selecting or dividing a compartment stream (3) into multiple compartment streams (3a, 3b), the fluid reservoir (47) has at least two outlets, from which one of the compartment streams (3a, 3b) is alternately sucked away with the flow rate ($\Phi4$, $\Phi8$) and pumps (71, 72) for sucking away the compartment streams (3a, 3b) are controlled with the aid of detectors (60) for detecting phase boundaries.

14. Method according to Claim 13, **characterized in that**, for determining a selection criterion for dividing the compartment stream (3), the fluid reservoir (47) includes an analysing device (69, 19), which is used for determining the state of the individual compartments.

15. Method according to Claim 10, **characterized in that**, for unifying compartments from two compartment streams (3a, 3b), the streams are fed to the ends of the fluid conductors (1, 2) of the surface-mounted probe (52) that are nested one inside the other, the united compartment stream being sucked away through the inner fluid conductor (1) and transport fluid (16) being fed in through the outer fluid conductor (2), and pumps for driving the compartment streams (3a, 3b) being controlled by detectors (60) for detecting phase boundaries in the compartment streams (3a, 3b).

16. Method according to Claim 10, **characterized in that**, for splitting a compartment into multiple small compartments, it is fed through a feed channel (78), the inside diameter of which corresponds to the outside diameter of the fluid conductor (2), directly to the ends of the fluid conductors (1, 2) that are nested one inside the other, at which ends the split compartments are sucked away through the inner fluid conductor (1) and transport liquid is fed in through the outer fluid conductor.

17. Method according to Claim 10, **characterized in that**, for metering an effector to the compartment, an effector (13) is fed to the outer fluid conductor (2), and **in that** detectors (60) for detecting phase boundaries in the compartment stream (3), consisting of compartments (25) and transport liquids (16), are used to control a pump (71), which is connected to a tube connection (31).

18. Method according to one of Claims 10 to 17, **characterized in that**, for imparting flows in the inner fluid conductor (1) and the outer fluid conductor (2), there are measuring devices for determining the actual flow, this actual flow is compared with a desired flow and the difference determined brings about a readjustment of the pumping output.

**Revendications**

1. Agencement de production, de manipulation et d'analyse de compartiments, comprenant au moins deux conducteurs de fluide (1, 2) pour guider des trajets de fluide séparés les uns des autres, **caractérisé en ce que** deux conducteurs de fluide (1, 2) sont disposés de manière montée l'un dans l'autre, une extrémité d'un premier conducteur de fluide (2) étant reliée à une source de flux pouvant être commandée pour imposer un flux d'un fluide de transport (16), une extrémité d'un deuxième conducteur de fluide (1) étant reliée à une source de flux pouvant être commandée supplémentaire pour imposer un flux d'un courant de compartiments (3) et les deux conducteurs de fluide (1, 2) étant plongés par leurs autres extrémités respectives dans un réservoir de fluide (4) dans lequel se trouve un fluide supplémentaire (15), lequel n'est pas miscible avec le fluide de transport (16).

2. Agencement selon la revendication 1, **caractérisé en ce que** le conducteur de fluide intérieur (1) est doté d'une surface frontale (5) à son extrémité plongeant dans le réservoir de fluide (4), laquelle surface frontale est complètement ou partiellement non mouillante pour le fluide (15) se trouvant dans le réservoir de fluide (4).

3. Agencement selon la revendication 1 ou 2, **caractérisé en ce que** la surface intérieure du conducteur de fluide (1) pour le courant de compartiments (3) est complètement ou partiellement non mouillante pour le fluide (15) se trouvant dans le réservoir de fluide.

4. Agencement selon l'une quelconque des revendications précédentes, **caractérisé en ce que** les conducteurs de fluide (1, 2) sont disposés dans une sonde amovible (50) qui comporte un corps de sonde (9) avec des raccords de tuyau séparés (31, 32) pour le courant de compartiments (3) et pour le fluide de transport (16).

5. Agencement selon l'une quelconque des revendications précédentes, **caractérisé en ce que** le réservoir de fluide (4) est réalisé sous forme de puits et des moyens d'étanchéité (37, 38) sont disposés sur la sonde amovible (50), au moyen desquels, après le placement de la sonde amovible (50) sur le puits (4), le fluide (15) dans le puits (4) est fermé de manière étanche aux gaz et/ou aux liquides vis-à-vis de l'environnement.

6. Agencement selon l'une quelconque des revendications 4 et 5, **caractérisé en ce que** des canaux de rinçage (33), (34) pour le guidage d'un fluide de rinçage (17) et/ou un canal d'aération (29) qui est fermé de manière stérile au moyen d'un filtre (30) est/sont disposé(s) dans le corps de sonde (9).

7. Agencement selon l'une quelconque des revendications 4 à 6, **caractérisé en ce que** des détecteurs (60) pour détecter les limites de phases dans le courant de compartiments (3) sont disposés dans la sonde amovible (50).

**8.** Agencement selon la revendication 7, **caractérisé en ce que** le réservoir de fluide (4) comporte plusieurs puits, au moins deux puits étant reliés au moyen de canaux de liaison (27) dans lesquels sont disposés des détecteurs (60, 60a, 60b) pour déterminer des limites de phases.

**9.** Agencement selon l'une quelconque des revendications 4 à 8, **caractérisé en ce que** le conducteur de fluide extérieur (2) dépasse par rapport au conducteur de fluide intérieur (1) d'une valeur $d_s$, le conducteur de fluide extérieur (2) est placé de manière étanche aux liquides sur le fond d'un puits (45), le puits (45) comporte une sortie (96) pour l'évacuation du courant de compartiments ajouté de manière dosée et **en ce que** des détecteurs (60, 62) pour détecter des limites de phases dans le courant de compartiments (3) sont présents.

**10.** Procédé de production, de manipulation et d'analyse de compartiments, **caractérisé en ce qu'**au moyen de deux conducteurs de liquide (1, 2) connectés l'un dans l'autre, qui pénètrent respectivement par une extrémité dans un réservoir de fluide (4) comprenant le fluide A, le flux ($\Phi$1) d'un fluide B est imposé au conducteur de fluide intérieur (1) et un flux ($\Phi$2) d'un fluide C est imposé au conducteur de fluide extérieur (2), les fluides A et B étant non miscibles.

**11.** Procédé selon la revendication 10, **caractérisé en ce que**, pour la production de compartiments, le réservoir de fluide (4) est rempli d'un fluide d'échantillon (15), le conducteur de fluide extérieur (2) est sollicité avec un flux ($\Phi$2) d'un fluide de transport (16) et un courant de compartiments (3) présentant un flux ($\Phi$1) est aspiré à travers le conducteur de fluide intérieur (1), le flux ($\Phi$1) étant supérieur au flux ($\Phi$2).

**12.** Procédé selon la revendication 10, **caractérisé en ce que**, pour décompartimenter le courant de compartiments (3) à travers le conducteur de fluide intérieur (1), un débit ($\Phi$1) s'écoule dans le réservoir de fluide (4), lequel comporte un fluide miscible avec les compartiments, et un liquide de transport (16) présentant le débit ($\Phi$2) est aspiré à travers le conducteur de fluide extérieur (2).

**13.** Procédé selon la revendication 10, **caractérisé en ce que**, pour sélectionner ou diviser un courant de compartiments (3) en plusieurs courants de compartiments (3a, 3b), le réservoir de fluide (47) comporte au moins deux sorties, hors desquelles l'un des courants de compartiments (3a, 3b) présentant le débit ($\Phi$4, $\Phi$8) est aspiré en alternance et, à l'aide de détecteurs (60) pour détecter des limites de phases dans le courant de compartiments (3), des pompes (71, 72) pour l'aspiration des courants de compartiments (3a, 3b) sont commandées.

**14.** Procédé selon la revendication 13, **caractérisé en ce que**, pour déterminer un critère de sélection pour diviser le courant de compartiments (3), le réservoir de fluide (47) comporte un dispositif d'analyse (69, 19) qui est utilisé pour déterminer l'état des compartiments individuels.

**15.** Procédé selon la revendication 10, **caractérisé en ce que**, pour combiner des compartiments de deux courant de compartiments (3a, 3b), ceux-ci sont acheminés jusqu'aux extrémités des conducteurs de fluide (1, 2) connectés l'un dans l'autre de la sonde amovible (52), le courant de compartiments combiné étant aspiré à travers le conducteur de fluide intérieur (1) et le fluide de transport (16) étant acheminé à travers le conducteur de fluide extérieur (2) et, au moyen de détecteurs (60) pour détecter des limites de phases dans le courant de compartiments (3a, 3b), des pompes pour l'entraînement des courants de compartiments (3a, 3b) étant commandées.

**16.** Procédé selon la revendication 10, **caractérisé en ce que**, pour fractionner un compartiment en plusieurs petits compartiments, celui-ci est acheminé, à travers un canal d'acheminement (78) dont le diamètre intérieur correspond au diamètre extérieur du conducteur de fluide (2), directement jusqu'aux extrémités des conducteurs de fluide (1, 2) connectés l'un dans l'autre, dans lesquels les compartiments fractionnés sont aspirés à travers le conducteur de fluide intérieur (1) et le liquide de transport est acheminé à travers le conducteur de fluide extérieur.

**17.** Procédé selon la revendication 10, **caractérisé en ce que**, pour ajouter de manière dosée un effecteur au compartiment, un effecteur (13) est acheminé jusqu'au conducteur de fluide extérieur (2), et **en ce que** des détecteurs (60) pour détecter des limites de phases dans le courant de compartiments (3) constitué de compartiments (25) et de liquides de transport (16) sont utilisés pour commander une pompe (71) qui est reliée à un raccord de tuyau (31).

**18.** Procédé selon l'une quelconque des revendications 10 à 17, **caractérisé en ce que**, pour imposer des flux dans le conducteur de fluide intérieur (1) et le conducteur de fluide extérieur (2), des dispositifs de mesure pour déterminer le flux réel sont présents, ce flux réel est comparé à un flux théorique et la différence déterminée provoque un réajustage de la puissance de pompage.

Fig. 1b

Fig. 1a

Fig. 2

Fig. 3a

Fig. 3b

Fig. 4

Fig. 5a

Fig. 5b

Fig. 6

Fig. 7

Fig. 8

Fig. 9

Fig. 10

Fig. 11
Stand der Technik

Fig. 12

Fig. 13

Fig. 14

Fig. 15

# Fig. 16

Fig. 17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10145568
- US 20080003142 A
- US 20050221339 A
- US 20050172476 A
- WO 2006098700 A
- DE 10322942
- DE 19715441
- US 5191900 A